# EUROPEAN PATENT APPLICATION

(11) **EP 4 527 936 A1**
(43) Date of publication of application: **26.03.2025**
(21) Application number: 23807026.2
(22) Date of filing: 18.05.2023
(51) Int. Cl.: C12P 17/14, C12N 1/38, C12N 1/14, C12R 1/645

(54) **METHOD FOR INCREASING FERMENTATION YIELD OF PF1022A**

(30) Priority: 20.05.2022 CN 202210560600
(71) Applicant: Zhejiang Hisun Pharmaceutical Co., Ltd., Taizhou, Zhejiang 318000 (CN)
(72) Inventor: XU, Qian, Taizhou, Zhejiang 318000 (CN); XIANG, Renxin, Taizhou, Zhejiang 318000 (CN); YANG, Xiaohu, Taizhou, Zhejiang 318000 (CN); FANG, Jiashuang, Taizhou, Zhejiang 318000 (CN); TAO, Liangliang, Taizhou, Zhejiang 318000 (CN); YING, Lingping, Taizhou, Zhejiang 318000 (CN); SUN, Peng, Taizhou, Zhejiang 318000 (CN); YANG, Zhiqing, Taizhou, Zhejiang 318000 (CN)
(74) Representative: Dehns
(86) International application number: PCT/CN2023/095010
(87) International publication number: WO 2023/222075

(57) **Abstract**

The present disclosure provides a method for increasing a fermentation yield of PF1022A, the method comprises taking a *Rosellinia sp.* capable of producing PF1022A as a production strain and performing aerobic fermentation in a fermentation medium, the fermentation medium comprises an assimilable carbon source, an assimilable nitrogen source and an inorganic salt, wherein the assimilable carbon source contains solid malt extract. The technical solution provided by the present disclosure is simple and easy to implement, improves the production efficiency of PF 1022A, greatly increases the output of PF 1022A, reduces the production costs, and facilitates large-scale industrial production of PF1022A.

## Description

### TECHNICAL FIELD

The present disclosure relates to the technical field of industrial microbial fermentation, and in particular to a method for increasing a fermentation yield of PF1022A.

### BACKGROUND

Emodepside is a semi-synthetic derivative with PF1022A as a precursor, which has killing effects on invertebrates (such as nematodes and roundworms). Bayer Animal Health has developed combinations of Emodepside with praziquantel (Profender) and with toltrazuril (Procox), respectively, for use as anthelmintics for cats and dogs, and Profender (in combination with praziquantel) went on sale in the United States in 2007. Bayer collaborated with DNDi to develop Emodepside (human medicine) for the treatment of "river blindness" caused by onchocerciasis, which entered the Phase 1 trial of healthy volunteers in 2016, completed a single ascending dose study in 2017, and completed a multiple ascending dose study at the end of 2018.

PF1022A, as a precursor raw material for the synthesis of Emodepside, has a very broad market prospect and has attracted much attention. At present, there are solid-phase synthesis and biosynthesis methods for the synthesis of PF1022A. The solid-phase synthesis method has cumbersome steps and complex processes, which is not suitable for large-scale production. However, there are few reports on the fermentation production of PF1022A, and the yield of PF1022A is relatively low. In view of this situation, our company has selected and bred a new strain of PF1022A-producing bacteria, which is specifically disclosed in CN201711276671.0. This strain has the advantages of high production capacity, high genetic stability and the like, but its disclosed fermentation method still has problems such as low fermentation yield, high fermentation cost and the like, which is not conducive to large-scale industrial production.

### SUMMARY

The present disclosure provides a method for increasing a fermentation yield of PF1022A. The method comprises: taking a *Rosellinia sp.* capable of producing PF1022A as a production strain and performing aerobic fermentation in a fermentation medium, the fermentation medium comprises an assimilable carbon source, an assimilable nitrogen source and an inorganic salt, wherein the assimilable carbon source contains solid malt extract.

In preferred embodiments, a content of the solid malt extract in the fermentation medium is 70-200 g/L, further preferably 100-200 g/L, more preferably 140-200 g/L, and most preferably 160 g/L.

In preferred embodiments, the assimilable carbon source further contains one selected from glucose, maltodextrin, maltose, soybean oil, soluble starch, corn starch, or a combination of any two or more of the above.

In preferred embodiments, the assimilable carbon source further contains soybean oil and maltodextrin, preferably soybean oil.

In preferred embodiments, the assimilable nitrogen source is selected from one of yeast powder, cottonseed cake powder, wheat germ powder, yeast extract powder, soybean cake powder, peanut cake powder, or a combination of any two or more of the above.

In preferred embodiments, the assimilable nitrogen source is a combination of yeast powder and cottonseed cake powder.

In preferred embodiments, the inorganic salt is selected from one of zinc sulfate, magnesium sulfate, ferrous sulfate, copper sulfate, manganese sulfate, diammonium hydrogen sulfate, potassium chloride, sodium chloride, magnesium chloride, nickel chloride, calcium carbonate, calcium chloride, or a combination of any two or more thereof.

In preferred embodiments, the inorganic salt is a combination of calcium carbonate, sodium chloride and magnesium sulfate.

In preferred embodiments, the fermentation medium contains: 75-210 g/L of the assimilable carbon source, 15-40 g/L of the assimilable nitrogen source, and 6-10 g/L of the inorganic salt; preferably, the fermentation medium contains: 105-210 g/L of the assimilable carbon source, 15-40 g/L of the assimilable nitrogen source, and 6-10 g/L of the inorganic salt; further preferably, the fermentation medium contains: 145-200 g/L of the assimilable carbon source, 15-40 g/L of the assimilable nitrogen source, and 6-10 g/L of the inorganic salt.

In preferred embodiments, the fermentation medium contains: solid malt extract, soybean oil, cottonseed cake powder, yeast powder, sodium chloride, magnesium sulfate and calcium carbonate.

In preferred embodiments, in the fermentation medium, soybean oil is 5-10 g/L, yeast powder is 5-10 g/L, cottonseed cake powder is 10-30 g/L, sodium chloride is 2-3 g/L, magnesium sulfate is 2-3 g/L, and calcium carbonate is 2-4 g/L.

In preferred embodiments, the fermentation medium contains 70-200 g/L of the solid malt extract, preferably 100-200 g/L, more preferably 140-200 g/L, and most preferably 160 g/L of the solid malt extract; 10 g/L of the soybean oil; 25 g/L of the cottonseed cake powder; 10 g/L of the yeast powder; 2 g/L of the sodium chloride; 2 g/L of the magnesium sulfate; and 2 g/L of the calcium carbonate.

In preferred embodiments, a culture temperature of the fermentation is 22-28°C, preferably 24-26°C, and more preferably 25°C; a culture time of the fermentation is 6-10 days, preferably 7-10 days, and most preferably 9 days.

In preferred embodiments, the *Rosellinia sp.* capable of producing PF1022A is *Rosellinia sp.* HS-NF-1412Z, with a deposit number of CGMCC NO. 13893.

The g/L mentioned in the present disclosure refers to the weight of related substances added to 1 L of the fermentation medium.

During the research process, after many experiments, the inventor unexpectedly found that: when the fermentation medium contains solid malt extract (especially within a certain range) as a carbon source, the fermentation yield of PF1022A can be significantly improved (the maximum fermentation yield can reach up to 6000 µg/mL or more); when it does not contain solid malt extract as a carbon source and the carbon source is other substances (such as corn starch, sucrose, maltose, etc.), this effect cannot be achieved. The technical solution provided by the present disclosure is simple and easy to implement, improves the production efficiency of PF1022A, greatly increases the output of PF1022A, reduces the production costs, and facilitates large-scale industrial production of PF1022A.

### DETAILED DESCRIPTION

The technical solution in the examples of the present disclosure is described below, and the described examples are only a part of the present disclosure. The following comparative examples and examples are used to illustrate the present disclosure, but cannot be used to limit the scope of the present disclosure. The experimental methods in the following examples without specifying specific conditions shall be selected according to conventional methods and conditions, or according to the product instructions.

In the present disclosure, the PF1022A-producing strain is HS-NF-1412Z, and the strain used in the examples of the present disclosure is *Rosellini sp.* HS-NF-1412Z, which is deposited in the China General Microbiological Culture Collection Center with a deposit number of CGMCC NO. 13893 and disclosed in CN201711276671.0. In the present disclosure, various materials and reagents described are materials and reagents commonly used in the field and can be obtained through conventional commercial channels.

High-performance liquid phase detection method for PF1022A: the chromatographic column used Elite C18 silica gel column (5 µm, 4.6*250 mm). The separation was carried out using acetonitrile:water (at a volume ratio of 80:20) as a mobile phase, with a flow rate of 1.0 mL/min, an ultraviolet wavelength of 218 nm, an injection volume of 20 µL, a column temperature of 30°C, the retention time of PF1022A was about 10.5 min, and the running time was 1.9 times the retention time of PF1022A.

### Preparation Example 1

(1) Preparation of slant culture medium: Potato dextrose agar (PDA) culture medium was used on the slant: 200 g of potatoes were cut into small pieces, added with 1000 mL of water and boiled for 30 min, the potato pieces were filtered out, the filtrate was made up to 1000 mL with water, added with 20 g of glucose and 18 g of agar, with natural pH, sterilized at 121°C for 20 min, poured into a plate when cooled to about 55°C, and cooled to solidify. The frozen strain numbered HS-NF-1412Z was inoculated into the slant culture medium and cultured at 25°C±1°C for 6 days.
(2) Preparation of seed culture medium: Each liter contained the following components: 20 g of glucose, 20 g of soluble corn starch, 15 g of yeast extract, 20 g of cottonseed cake powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20min.

Culture method for seed liquid: the fresh strain of *Roselliniia sp.* HS-NF-1412Z growing on the slant was inoculated into the seed culture medium, cultured at 25°C±1°C with a shaking speed of 250 r/min for 80 h.

### Comparative Example 1 - The carbon source contained corn starch (the content of corn starch in the fermentation medium was 120 g/L)

The seed liquid prepared in Preparation Example 1 was inoculated into a fermentation medium (1 L) containing the following ingredients: 120 g of corn starch with 0.024 g amylase for pre-gelatinization, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days.

PF1022A was detected in the fermentation solution as follows (same for the detection method for PF1022A in Comparative Examples 2-4 and Examples 1-9):
① the fermentation broth was added with anhydrous methanol to dilute 5 times and shaken well;
② ultrasonication was performed for 30 min, and the ultrasonic fermentation broth was filtered;
③ the supernatant was taken and filtered with a 0.22 µm organic phase needle filter;
④ the filtered liquid was detected by HPLC, and the fermentation unit of PF1022A was 3290 µg/mL.

### Comparative Example 2 - The carbon source contained maltodextrin (the content of maltodextrin in the fermentation medium was 120 g/L)

The seed liquid prepared in Preparation Example 1 was inoculated into a fermentation medium (1 L) containing the following ingredients: 120 g of maltodextrin, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 3112 µg/mL.

### Comparative Example 3 - The carbon source contained maltose (the content of maltose in the fermentation medium was 120 g/L)

The seed liquid prepared in Preparation Example 1 was inoculated into a fermentation medium (1 L) containing the following ingredients: 120 g of maltose, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 3213 µg/mL.

### Comparative Example 4 - The carbon source contained saccharose (the content of saccharose in the fermentation medium was 120 g/L)

The seed liquid prepared in Preparation Example 1 was inoculated into a fermentation medium (1 L) containing the following ingredients: 120 g of saccharose, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 623 µg/mL.

### Example 1 - The carbon source contained solid malt extract (the content of solid malt extract in the fermentation medium was 120 g/L)

The seed liquid prepared in Preparation Example 1 was inoculated into a fermentation medium (1 L) containing the following ingredients: 120 g of solid malt extract, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 5540 µg/mL.

### Example 2 - The carbon source contained solid malt extract (the content of solid malt extract in the fermentation medium was 70 g/L)

The seed liquid prepared in Preparation Example 1 was inoculated into a fermentation medium (1 L) containing the following ingredients: 70 g of solid malt extract, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 4112 µg/mL.

### Example 3 - The carbon source contained solid malt extract (the content of solid malt extract in the fermentation medium was 100 g/L)

The seed liquid prepared in Preparation Example 1 was inoculated into a fermentation medium (1 L) containing the following ingredients: 100 g of solid malt extract, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 4905 µg/mL.

### Example 4 - The carbon source contained solid malt extract (the content of solid malt extract in the fermentation medium was 140 g/L)

The seed liquid prepared in Preparation Example 1 was inoculated into a fermentation medium (1 L) containing the following ingredients: 140 g of solid malt extract, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 5870 µg/mL.

### Example 5 - The carbon source contained solid malt extract

The seed liquid prepared in Preparation Example 1 was inoculated into a fermentation medium (1 L) containing the following ingredients: 140 g of solid malt extract, 10 g of soybean oil, 25 g of soybean cake powder, 10 g of yeast extract powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 5562 µg/mL.

### Example 6 - The carbon source contained solid malt extract

The seed liquid prepared in Preparation Example 1 was inoculated into a fermentation medium (1 L) containing the following ingredients: 140 g of solid malt extract, 40 g of maltodextrin, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 5412 µg/mL.

### Example 7 - The carbon source contained solid malt extract (the content of solid malt extract in the fermentation medium was 160 g/L)

The seed liquid prepared in Preparation Example 1 was inoculated into a fermentation medium (1 L) containing the following ingredients: 160 g of solid malt extract, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. The content of PF1022A in the fermentation broths shaking cultured in a shaking table at 25°C and 250 r/min for different culture times (6-10 days) was detected. The fermentation unit results of PF1022A determined by HPLC are shown in Table 1. The yield of PF1022A was higher when the fermentation culture time was 6-10 days, especially on the 9th day, the maximum yield of PF1022A was 6148 µg/mL.

**Table 1. Fermentation units of PF1022A determined by HPLC under different culture times**

| Culture days | 6 Days | 7 Days | 8 Days | 9 Days | 10 Days |
|---|---|---|---|---|---|
| Fermentation unit (µg/mL) | 4108 | 5312 | 5747 | 6148 | 6017 |

### Example 8 - The carbon source contained solid malt extract (the content of solid malt extract in the fermentation medium was 180 g/L)

The seed liquid prepared in Preparation Example 1 was inoculated into a fermentation medium (1 L) containing the following ingredients: 180 g of solid malt extract, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF 1022A was 5900 µg/mL.

### Example 9 - The carbon source contained solid malt extract (the content of solid malt extract in the fermentation medium was 200 g/L)

The seed liquid prepared in Preparation Example 1 was inoculated into a fermentation medium (1 L) containing the following ingredients: 200 g of solid malt extract, 10 g of soybean oil, 25 g of cottonseed cake powder, 10 g of yeast powder, 2 g of sodium chloride, 2 g of magnesium sulfate, and 2 g of calcium carbonate. The above substances were added with water to make up to 1000 mL, with pH 6.0, and a 250 mL triangular shake flask was used, filled with 25 mL, and sterilized at 121°C for 20 min. A fermentation broth was obtained by shake cultivation in a shaking table at 25°C and 250 r/min for 9 days. The fermentation broth was detected by HPLC, and the yield of PF1022A was 5743 µg/mL.

## Claims

1. A method for increasing a fermentation yield of PF1022A, wherein, the method comprises: taking a *Rosellinia sp.* capable of producing PF1022A as a production strain and performing aerobic fermentation in a fermentation medium, the fermentation medium comprises an assimilable carbon source, an assimilable nitrogen source and an inorganic salt, wherein the assimilable carbon source contains solid malt extract.

2. The method according to claim 1, wherein, a content of the solid malt extract in the fermentation medium is 70-200 g/L, further preferably 100-200 g/L, more preferably 140-200 g/L, and most preferably 160 g/L.

3. The method according to claim 1 or 2, wherein, the assimilable carbon source further contains one selected from glucose, maltodextrin, maltose, soybean oil, soluble starch, corn starch, or a combination of any two or more of the above; preferably soybean oil and maltodextrin, and more preferably soybean oil.

4. The method according to any one of claims 1-3, wherein, the assimilable nitrogen source is selected from one of yeast powder, cottonseed cake powder, wheat germ powder, yeast extract powder, soybean cake powder, peanut cake powder, or a combination of any two or more of the above, preferably a combination of yeast powder and cottonseed cake powder.

5. The method according to any one of claims 1-4, wherein, the inorganic salt is selected from one of zinc sulfate, magnesium sulfate, ferrous sulfate, copper sulfate, manganese sulfate, diammonium hydrogen sulfate, potassium chloride, sodium chloride, magnesium chloride, nickel chloride, calcium carbonate, calcium chloride, or a combination of any two or more thereof, preferably a combination of calcium carbonate, sodium chloride and magnesium sulfate.

6. The method according to any one of claims 1-5, wherein, the fermentation medium contains: 75-210 g/L of the assimilable carbon source, 15-40 g/L of the assimilable nitrogen source, and 6-10 g/L of the inorganic salt.

7. The method according to any one of claims 1-6, wherein, the fermentation medium contains: solid malt extract, soybean oil, cottonseed cake powder, yeast powder, sodium chloride, magnesium sulfate and calcium carbonate.

8. The method according to claim 7, wherein, soybean oil is 5-10 g/L, yeast powder is 5-10 g/L, cottonseed cake powder is 10-30 g/L, sodium chloride is 2-3 g/L, magnesium sulfate is 2-3 g/L, and calcium carbonate is 2-4 g/L.

9. The method according to any one of claims 1-8, wherein, a culture temperature of the fermentation is 22-28°C, preferably 24-26°C; a culture time of the fermentation is 6-10 days, preferably 7-10 days, and most preferably 9 days.

10. The method according to any one of claims 1-9, wherein, the *Rosellinia sp.* capable of producing PF1022A is *Rosellinia sp.* HS-NF-1412Z, with a deposit number of CGMCC NO. 13893.
